# FASCICULE DE BREVET EUROPEEN

(11) **EP 1 038 857 B1**
(45) Date de publication et mention de la délivrance du brevet: **18.08.2004**
(21) Numéro de dépôt: 00200976.9
(22) Date de dépôt: 16.03.2000
(51) Int. Cl.: C07C 19/08, C07C 17/38, C07C 17/383, C07C 17/20

(54) **Procédé pour la séparation de mélanges comprenant du fluorure d'hydrogène et du 1,1,1,3,3-pentafluorobutane et procédé pour la synthèse de 1,1,1,3,3-pentafluorobutane**
Verfahren zur Trennung von Fluorwasserstoff und 1,1,1,3,3-Pentafluorbutan enthaltenden Gemischen und Verfahren zur Herstellung von 1,1,1,3,3-Pentafluorbutan
Process for the separation of mixtures containing hydrogen fluoride and 1,1,1,3,3-pentafluorobutane and process for the preparation of 1,1,1,3,3-pentafluorobutane

(30) Priorité: 24.03.1999 FR 9903918
(43) Date de publication de la demande: 27.09.2000
(73) Titulaire: SOLVAY (Société Anonyme), 1050 Bruxelles (BE)
(72) Inventeur: Reif, Ferdinand, 1020 Bruxelles (BE); Balthasart, Dominique, 1120 Bruxelles (BE)
(74) Mandataire: Jacques, Philippe

(56) Documents cités:
- EP-A- 0 699 649
- EP-A- 0 885 863
- WO-A-97/13719
- US-A- 5 948 381

## Description

La présente invention concerne un procédé pour la séparation de mélanges comprenant éventuellement des compositions azéotropiques ou pseudo-azéotropiques contenant du fluorure d'hydrogène et du 1,1,1,3,3-pentafluorobutane et un procédé pour la synthèse de 1,1,1,3,3-pentafluorobutane

Le 1,1,1,3,3-pentafluorobutane (HFC-365mfc) peut être préparé par réaction d'un précurseur chloré approprié avec du fluorure d'hydrogène, tel que décrit par exemple dans la demande de brevet EP-A1-0699649 au nom de SOLVAY. Dans un tel procédé, à la sortie du réacteur d'hydrofluoration, le mélange de produits réactionnels contient, outre le 1,1,1,3,3-pentafluorobutane recherché, du chlorure d'hydrogène provenant de l'élimination du ou des atomes de chlore du précurseur chloré de départ, du fluorure d'hydrogène et, éventuellement, des diluants inertes ainsi que divers intermédiaires ou sous-produits en faibles quantités. Etant donné que l'on travaille habituellement avec un excès de fluorure d'hydrogène par rapport au précurseur chloré, il subsiste le plus souvent du fluorure d'hydrogène non converti dans le mélange de produits réactionnels. WO 97/13719 divulgue la séparation de mélanges comprenant du fluorure d'hydrogène et un composé organique par un procédé comprenant une étape d'extraction avec un fluorure alcalin et une étape dans laquelle du fluorure d'hydrogène est récupéré.

Il est souhaitable de disposer de procédés qui permettent de séparer efficacement le 1,1,1,3,3-pentafluorobutane du mélange de produits réactionnels. Il est en même temps souhaitable d'assurer une bonne utilisation de matières premières, notamment du fluorure d'hydrogène.

En conséquence, l'invention concerne un procédé pour la séparation d'au moins un constituant d'un mélange comprenant du 1,1,1,3,3-pentafluorobutane et de fluorure d'hydrogène, selon lequel on soumet le mélange à au moins deux distillations, la première distillation étant effectuée à une première pression et la deuxième distillation étant effectuée à une deuxième pression différente de la première, et on récupère au moins une fraction enrichie en 1,1,1,3,3-pentafluorobutane et au moins une fraction enrichie en fluorure d'hydrogène. On a trouvé, de manière surprenante, que le procédé selon l'invention permet d'isoler du 1,1,1,3,3-pentafluorobutane et du fluorure d'hydrogène essentiellement purs, en particulier à partir de mélanges comprenant du 1,1,1,3,3-pentafluorobutane et du fluorure d'hydrogène dans des proportions dans lesquelles ils forment un azéotrope ou un pseudo-azéotrope.

Le procédé selon l'invention permet également d'isoler des fractions azéotropiques à une pression donnée, enrichies soit en fluorure d'hydrogène soit en 1,1,1,3,3-pentafluorobutane.

Dans une variante du procédé selon l'invention la première distillation s'effectue à une pression plus élevée et la deuxième distillation s'effectue à une pression moins élevée.

Dans une autre variante du procédé selon l'invention la première distillation s'effectue à une pression moins élevée et la deuxième distillation s'effectue à une pression plus élevée.

La pression moins élevée à utiliser dans le procédé selon l'invention est généralement d'au moins 0,5 bar. Souvent cette pression est d'au moins 0,9 bar. Généralement la pression moins élevée est d'au plus 3 bar. Souvent cette pression est d'au plus 2,5 bar. De préférence la pression moins élevée est de 1 à 2 bar.

La température à laquelle s'effectue la distillation à pression moins élevée est généralement d'au moins 0°C. La température est souvent d'au moins 10°C. La température est généralement d'au plus 60°C. Souvent, la température est d'a plus 40°C. De préférence, la température à laquelle s'effectue la distillation à pression moins élevée est de 15 à 30°C.

La pression plus élevée à utiliser dans le procédé selon l'invention est généralement d'au moins 6 bar. Souvent cette pression est d'au moins 7 bar. Généralement la pression plus élevée est d'au plus 12 bar. Souvent cette pression est d'au plus 11 bar. De préférence la pression plus élevée est de 8 à 10 bar.

La température à laquelle s'effectue la distillation à pression plus élevée est généralement d'au moins 70°C. La température est souvent d'au moins 80°C. La température est généralement d'au plus 150°C. Souvent, la température est d'a plus 120°C. De préférence, la température à laquelle s'effectue la distillation à pression plus élevée est de 80 à 100°C.

La teneur en 1,1,1,3,3-pentafluorobutane dans la fraction enrichie en 1,1,1,3,3-pentafluorobutane est souvent d'au moins 50% molaire. Plus souvent elle est d'au moins 75% molaire. Dans une variante préférée, on récupère une fraction enrichie constituée essentiellement de 1,1,1,3,3-pentafluorobutane, par exemple avec une teneur en 1,1,1,3,3-pentafluorobutane d'au moins 99% molaire.

La teneur en fluorure d'hydrogène dans la fraction enrichie en fluorure d'hydrogène peut être d'au moins 99% molaire. Elle peut être d'au moins 99,5% molaire. Dans une variante, on récupère une fraction enrichie constituée essentiellement de fluorure d'hydrogène.

Des techniques et appareillages utilisables pour effectuer les distillations dans le procédé selon l'invention sont bien connus.

Le mélange auquel on applique le procédé de séparation selon l'invention peut être, par exemple, un mélange réactionnel issu d'un procédé de synthèse de 1,1,1,3,3-pentafluorobutane par hydrofluoration d'un précurseur chloré tel que par exemple le procédé mentionné plus haut. La procédé s'applique souvent à des mélanges comprenant le fluorure d'hydrogène et le 1,1,1,3,3-pentafluorobutane dans des proportions dans lesquelles ils forment un azéotrope ou un pseudo-azéotrope.

Dans une variante du procédé selon l'invention, on met en oeuvre à titre de mélange un mélange contenant une composition azéotropique constituée essentiellement de 1,1,1,3,3-pentafluorobutane et de fluorure d'hydrogène et comprenant éventuellement en outre du chlorure d'hydrogène, des chloro(fluoro)butanes de formule générale (I)

CClₐF₃₋ₐCH₂CCl_{b}F_{2-b}CH₃ (I)

avec a un entier de 0 à 3, b un entier de 0 à 2, la somme de a et b étant au moins égale à 1 ou de faibles quantités d'autres produits organiques.

Fondamentalement, l'état thermodynamique d'un fluide est défini par quatre variables interdépendantes : la pression (P), la température (T), la composition de la phase liquide (X) et la composition de la phase gazeuse (Y). Un azéotrope vrai est un système particulier à 2 ou plusieurs composants pour lequel, à une température donnée et à une pression donnée, la composition de la phase liquide X est exactement égale à la composition de la phase gazeuse Y. Un pseudo-azéotrope est un système à 2 ou plusieurs composants pour lequel, à une température donnée et à une pression donnée, X est substantiellement égal à Y. En pratique, cela signifie que les constituants de tels systèmes azéotropiques et pseudo-azéotropiques ne peuvent pas être séparés facilement par distillation

Aux fins de la présente invention, on entend par mélange pseudo-azéotropique, un mélange de deux constituants dont le point d'ébullition (à une pression donnée) diffère du point d'ébullition de l'azéotrope vrai de 0,5°C au maximum. Les mélanges dont le point d'ébullition diffère du point d'ébullition de l'azéotrope vrai de 0,2°C au maximum sont préférés. Les mélanges dont le point d'ébullition diffère du point d'ébullition de l'azéotrope vrai de 0,1 °C au maximum sont particulièrement préférés.

A une pression de 3 bar, la composition du mélange azéotropique fluorure d'hydrogène/1,1,1,3,3-pentafluorobutane est d'environ 60/40 % en poids, soit un rapport molaire fluorure d'hydrogène/1,1,1,3,3-pentafluorobutane d'environ 11 mol/mol.

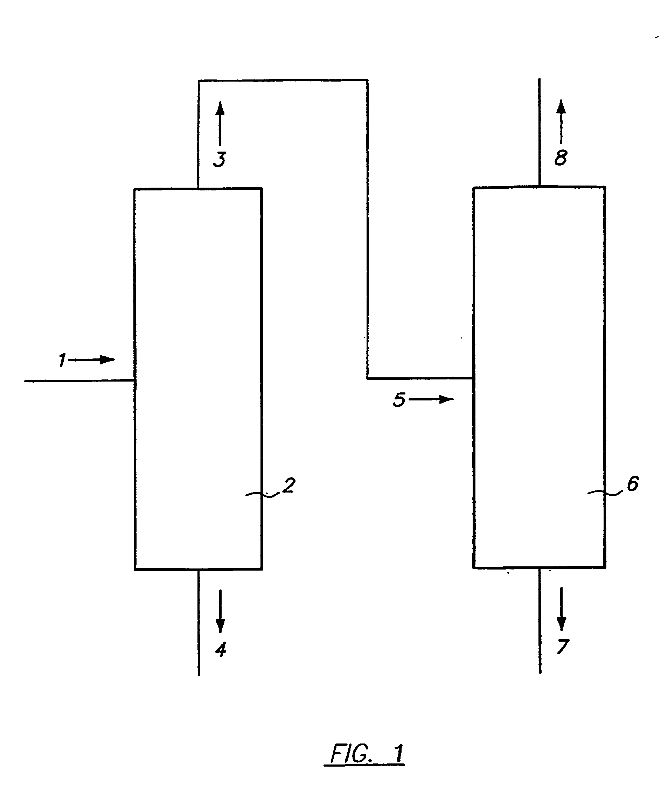

La figure 1 représente un schéma d'installation utilisable pour mettre en oeuvre le procédé de séparation selon l'invention.

Dans la première variante du procédé selon l'invention la première distillation s'effectue à une pression plus élevée et la deuxième distillation s'effectue à une pression moins élevée. Les numéros font référence à la figure 1. Dans cette variante, le mélange est introduit par la voie (1) dans une première colonne de distillation (2) à pression plus élevée. En tête (3) de cette colonne (2) on obtient une composition azéotropique de 1,1,1,3,3-pentafluorobutane et fluorure d'hydrogène dont la composition est déterminée par la pression dans la colonne (2). En pied (4) de cette colonne (2) on obtient une fraction contenant du 1,1,1,3,3-pentafluorobutane ou du fluorure d'hydrogène, selon que l'un ou l'autre de ces composés est en excès dans le mélange introduit par la voie (1) par rapport à la composition de l'azéotrope soutiré en tête (3), ainsi que les composés organiques à point d'ébullition plus élevé que l'azéotrope éventuellement présents dans le mélange à séparer, tels que des chloro(fluoro)butanes de formule générale (I). La composition récoltée en tête (3) de la colonne (2) est introduite par la voie (5) dans la deuxième colonne de distillation (6) à pression moins élevée. On récupère, en pied (7) de la colonne (6), une fraction enrichie en 1,1,1,3,3-pentafluorobutane par rapport à la composition azéotropique récupérée en (3). De préférence on récupère en (7) une fraction constituée essentiellement de 1,1,1,3,3-pentafluorobutane. En tête (8) de la deuxième colonne (6), on obtient une composition azéotropique, riche en fluorure d'hydrogène par rapport à la composition azéotropique récupérée en (3).

Dans la deuxième variante du procédé selon l'invention la première distillation s'effectue à une pression moins élevée et la deuxième distillation s'effectue à une pression plus élevée. Dans cette variante, on récupère, en pied (7) de la deuxième colonne (6), une fraction enrichie en fluorure d'hydrogène. De préférence, on récupère une fraction constituée essentiellement de fluorure d'hydrogène. En tête (8) de la deuxième colonne (6), on obtient une composition azéotropique riche en 1,1,1,3,3-pentafluorobutane.

L'invention concerne aussi en particulier un procédé pour la synthèse de 1,1,1,3,3-pentafluorobutane comprenant au moins les étapes (a) à (d) suivantes:
(a) on soumet des chloro(fluoro)butanes de formule générale (I) à au moins une réaction d'hydrofluoration;
(b) on soumet le produit de la réaction comprenant au moins du 1,1,1,3,3-pentafluorobutane et du fluorure d'hydrogène à une opération de séparation, généralement une distillation, pour séparer le chlorure d'hydrogène d'un mélange comprenant au moins du 1,1,1,3,3-pentafluorobutane et du fluorure d'hydrogène et éventuellement d'autres constituants du produit de la réaction;
(c) on soumet ledit mélange au procédé de séparation selon l'invention;
(d) on introduit au moins une fraction récupérée à l'étape (c), comprenant du fluorure d'hydrogène et éventuellement des autres constituants du produit de la réaction dans une étape d'hydrofluoration.

Dans un mode préféré du procédé de synthèse selon l'invention, on utilise à l'étape (c) la variante du procédé de séparation discutée plus haut, dans laquelle la première distillation s'effectue à une pression plus élevée et la deuxième distillation à une pression moins élevée. Dans ce cas, on renvoie avantageusement à l'étape (a) le flux récupéré en pied de la première colonne de distillation, constitué généralement de fluorure d'hydrogène excédentaire par rapport à la composition azéotropique soutirée en tête de la première colonne de distillation et de chloro(fluoro)butanes de formule générale (I), ainsi que le flux récupéré en tête de la deuxième colonne de distillation, constitué essentiellement d'une composition azéotropique selon l'invention riche en fluorure d'hydrogène.

Souvent le mélange séparé à l'étape (b) et soumis au procédé de séparation à l'étape (c) contient des chloro(fluoro)butanes de formule générale (I) à titre d'autres constituants du produit de la réaction.

La réaction d'hydrofluoration de l'étape (a) peut être catalytique ou non catalytique.

De préférence, le procédé de synthèse selon l'invention met en oeuvre un précurseur chloré du 1,1,1,3,3-pentafluorobutane. De manière particulièrement préférée, le précurseur chloré est du 1,1,1,3,3-pentachlorobutane.

## Revendications

1. Procédé pour la séparation d'au moins un constituant d'un mélange comprenant du 1,1,1,3,3-pentafluorobutane et du fluorure d'hydrogène, selon lequel on soumet le mélange à au moins deux distillations, la première distillation étant effectuée à une première pression et la deuxième distillation étant effectuée à une deuxième pression différente de la première, et on récupère au moins une fraction enrichie en 1,1,1,3,3-pentafluorobutane et au moins une fraction enrichie en fluorure d'hydrogène.

2. Procédé selon la revendication 1, dans lequel la première distillation s'effectue à une pression plus élevée et la deuxième distillation s'effectue à une pression moins élevée.

3. Procédé selon la revendication 2 selon lequel on récupère une fraction constituée essentiellement de 1,1,1,3,3-pentafluorobutane en pied de la deuxième distillation.

4. Procédé selon la revendication 2 ou 3, dans lequel on soutire en tête de la première distillation une composition azéotropique de 1,1,1,3,3 pentafluorobutane et de fluorure d'hydrogène que l'on introduit dans la deuxième distillation.

5. Procédé selon la revendication 1, dans lequel la première distillation s'effectue à une pression moins élevée et la deuxième distillation s'effectue à une pression plus élevée.

6. Procédé selon la revendication 4 selon lequel on récupère une fraction constituée essentiellement de fluorure d'hydrogène en pied de la deuxième distillation.

7. Procédé selon l'une quelconque des revendications 2 à 5 dans lequel la pression moins élevée est de 0,5 bar à 3 bar et la pression plus élevée est de 6 bar à 11 bar.

8. Procédé selon l'une quelconque des revendications 2 à 6 dans lequel la température à laquelle on effectue la distillation à pression moins élevée est de 0°C à 60°C et la température à laquelle on effectue la distillation à pression plus élevée est de 70°C à 150°C.

9. Procédé pour la synthèse de 1,1,1,3,3-pentafluorobutane comprenant au moins les étapes (a) à (d) suivantes :
(a) on soumet des chloro(fluoro)butanes de formule générale (I)
CClₐF₃₋ₐCH₂CCl_{b}F_{2-b}CH₃ (I)
avec a un entier de 0 à 3, b un entier de 0 à 2, la somme de a et b étant au moins égale à 1 à au moins une réaction d'hydrofluoration;
(b) on soumet le produit de la réaction comprenant au moins du 1,1,1,3,3-pentafluorobutane et du fluorure d'hydrogène à une opération de séparation, pour séparer le chlorure d'hydrogène d'un mélange comprenant au moins du 1,1,1,3,3-pentafluorobutane et du fluorure d'hydrogène et éventuellement d'autres constituants du produit de la réaction;
(c) on soumet ledit mélange au procédé de séparation selon une quelconque des revendications 1 à 8;
(d) on introduit au moins une fraction récupérée à l'étape (c), comprenant du fluorure d'hydrogène et éventuellement des autres constituants du produit de la réaction dans une étape d'hydrofluoration.

10. Procédé selon l'une quelconque des revendications 1 à 9, dans lequel le mélange comprend le fluorure d'hydrogène et le 1,1,1,3,3-pentafluorobutane dans des proportions dans lesquelles ils forment un azéotrope ou un pseudo-azéotrope.

## Patentansprüche

1. Verfahren zur Abtrennung wenigstens eines Bestandteiles eines Gemisches, das 1,1,1,3,3 Pentafluorbutan und Fluorwasserstoff umfaßt, wonach das Gemisch wenigstens zwei Destillationen unterzogen wird, wobei die erste Destillation bei einem ersten Druck ausgeführt wird und die zweite Destillation bei einem zweiten, vom ersten Druck verschiedenen Druck ausgeführt wird und wenigstens eine an 1, 1, 1,3,3 -Pentafluorbutan angereicherte Fraktion und wenigstens eine an Fluorwasserstoff angereicherte Fraktion gewonnen werden.

2. Verfahren nach Anspruch 1, worin die erste Destillation bei einem höheren Druck ausgeführt wird und die zweite Destillation bei einem weniger hohen Druck ausgeführt wird.

3. Verfahren nach Anspruch 2, wonach eine im wesentlichen aus 1,1,1,3,3-Pentafluorbutan bestehende Fraktion am Sumpf der zweiten Destillation gewonnen wird.

4. Verfahren nach Anspruch 2 oder 3, wonach am Kopf der ersten Destillation eine azeotrope Zusammensetzung aus 1,1,1,3,3-Pentafluorbutan und Fluorwasserstoff abgezogen wird, die in die zweite Destillation eingeführt wird.

5. Verfahren nach Anspruch 1, worin die erste Destillation bei einem weniger hohen Druck ausgeführt wird und die zweite Destillation bei einem höheren Druck ausgeführt wird.

6. Verfahren nach Anspruch 4, wonach eine im wesentlichen aus Fluorwasserstoff bestehende Fraktion am Sumpf der zweiten Destillation gewonnen wird.

7. Verfahren nach einem der Ansprüche 2 bis 5, worin der weniger hohe Druck 0,5 bar bis 3 bar beträgt und der höhere Druck 6 bar bis 11 bar beträgt.

8. Verfahren nach einem der Ansprüche 2 bis 6, worin die Temperatur, bei der die Destillation bei weniger hohem Druck vorgenommen wird, 0°C bis 60°C beträgt und die Temperatur, bei der die Destillation bei höherem Druck vorgenommen wird, 70°C bis 150°C beträgt.

9. Verfahren zur Synthese von 1,1,1,3,3-Pentafluorbutan, das zumindest die nachfolgenden Schritte (a) bis (d) umfaßt:
(a) Chlor(fluor)butane der allgemeinen Formel (I)
CClₐF₃₋ₐCH₂CCl_{b}F_{2-b}CH₃ (I),
worin a eine ganze Zahl von 0 bis 3 bedeutet, b eine ganze Zahl von 0 bis 2 ist und die Summe aus a und b wenigstens 1 beträgt, werden wenigstens einer Hydrofluorierungsreaktion unterzogen;
(b) das Reaktionsprodukt, das wenigstens 1,1,1,3,3-Pentafluorbutan und Fluorwasserstoff umfaßt, wird einer Trennoperation unterzogen, um Chlorwasserstoff von einem Gemisch abzutrennen, das zumindest 1,1,1,3,3-Pentafluorbutan und Fluorwasserstoff und gegebenenfalls weitere Bestandteile des Reaktionsproduktes umfaßt;
(c) dieses Gemisch wird dem Trennverfahren nach einem der Ansprüche 1 bis 8 unterworfen;
(d) wenigstens eine im Schritt (c) gewonnene Fraktion, die Fluorwasserstoff und gegebenenfalls weitere Bestandteile des Reaktionsproduktes umfaßt, wird in eine Hydrofluorierungsstufe eingeführt.

10. Verfahren nach einem der Ansprüche 1 bis 9, worin das Gemisch den Fluorwasserstoffund das 1,1,1,3,3-Pentafluorbutan in Verhältnissen enthält, unter denen sie ein Azeotrop oder ein Pseudoazeotrop ausbilden.

## Claims

1. Process for the separation of at least one constituent from a mixture comprising 1,1,1,3,3-pentafluorobutane and hydrogen fluoride, according to which the mixture is subjected to at least two distillations, the first distillation being carried out at a first pressure and the second distillation being carried out at a second pressure which is different from the first, and at least one fraction enriched in 1,1,1,3,3-pentafluorobutane and at least one fraction enriched in hydrogen fluoride are recovered.

2. Process according to Claim 1, in which the first distillation is carried out at a higher pressure and the second distillation is carried out at a lower pressure.

3. Process according to Claim 2 according to which a fraction consisting essentially of 1,1,1,3,3-pentafluorobutane is recovered at the foot of the second distillation.

4. Process according to Claim 2 or 3, in which the top of the first distillation, an azeotropic composition of 1, 1,1,3,3-pentafluorobutane and hydrogen fluoride is withdrawn, which is introduced into the second distillation.

5. Process according to Claim 1, in which the first distillation is carried out at a lower pressure and the second distillation is carried out at a higher pressure.

6. Process according to Claim 4 according to which a fraction consisting essentially of hydrogen fluoride is recovered at the foot of the second distillation.

7. Process according to any one of Claims 2 to 5 in which the lower pressure is 0.5 bar to 3 bar and the higher pressure is 6 bar to 11 bar.

8. Process according to any one of Claims 2 to 6 in which the temperature at which the distillation at lower pressure is carried out is 0°C to 60°C and the temperature at which the distillation at higher pressure is carried out is 70°C to 150°C.

9. Process for the synthesis of 1,1,1,3,3-pentafluorobutane comprising at least the following steps (a) to (d):
(a) chloro(fluoro)butanes of general formula (I)
CClₐF₃₋ₐCH₂CCl_{b}F_{2-b}CH₃ (I)
with a an integer of 0 to 3, b an integer of 0 to 2, the sum of a and b being at least equal to 1 are subjected to at least one hydrofluorination reaction;
(b) the reaction product comprising at least 1,1,1,3,3-pentafluorobutane and hydrogen fluoride is subjected to a separation procedure, generally a distillation, to separate the hydrogen chloride from a mixture which comprises at least 1,1,1,3,3-pentafluorobutane and hydrogen fluoride and optionally other constituents of the reaction product;
(c) the said mixture is subjected to the process of separation according to any one of claims 1 to 8;
(d) at least one fraction recovered in step (c), comprising hydrogen fluoride and optionally other constituents of the reaction product, is introduced into a hydrofluorination step.

10. Process or method according to any one of Claims 1 to 9, in which the mixture comprises hydrogen fluoride and 1,1,1,3,3-pentafluorobutane in proportions at which they form an azeotrope or a pseudoazeotrope.
